# EUROPEAN PATENT SPECIFICATION

(11) **EP 4 093 301 B1**
(45) Date of publication and mention of the grant of the patent: **22.04.2026**
(21) Application number: 20915946.6
(22) Date of filing: 16.12.2020
(51) Int. Cl.: A61B 17/17, A61B 17/04, A61B 17/00

(54) **SELF-POSITIONING DRILL GUIDE**
SELBSTPOSITIONIERENDE BOHRFÜHRUNG
GUIDE DE FORAGE À POSITIONNEMENT AUTOMATIQUE

(30) Priority: 23.01.2020 US 202016750259
(43) Date of publication of application: 30.11.2022
(60) Divisional application: 25220965.5
(73) Proprietor: Arthrex, Inc., Naples, FL 34108-1945 (US)
(72) Inventor: CAMPAGNOLI, Alexander, 85609 Aschheim (DE); KEUPER, Ronja, 81675 Munchen (DE)
(74) Representative: Lohr, Jöstingmeier & Partner Patent- und Rechtsanwälte mbB
(86) International application number: PCT/US2020/065187
(87) International publication number: WO 2021/150325

(56) References cited:
- US-A- 5 817 098
- US-A1- 2006 074 434
- US-A1- 2007 233 136
- US-A1- 2009 254 093
- US-A1- 2011 137 341
- US-A1- 2011 270 255
- US-A1- 2012 136 357
- US-A1- 2014 058 466
- US-A1- 2016 022 255
- US-A1- 2017 181 757
- US-A1- 2019 290 298

## Description

### BACKGROUND

The present disclosure relates to the field of surgery and, more particularly, to self-positioning drill guides and methods of surgical repairs. US 2014/0058466 Al discloses surgical guiding tools and systems.

### SUMMARY

The drill guide according to the invention is defined in claim 1. Further embodiments of the invention are recited in the dependent claims.

Surgical constructs, assemblies, and kits are disclosed. A drill guide is configured to automatically reference a drill point or drill angle for drilling tunnels in bones by using peripheral edges. A drill guide can be a positioning drill guide. A drill guide can be a self-positioning drill guide. The automatic positioning may occur at the center of the bone, but the positioning may also occur at any other desired location on the bone. A self-positioning drill guide can be a center point drill guide configured to automatically center drill tunnels in bones by using peripheral edges of the bone that precisely reference the center point. The centering guide helps to automatically determine the center of peripheral bone edges as well as to provide increased control during drilling.

Methods of surgeries are also disclosed. In an embodiment, a method of positioning and drilling a bone tunnel in a bone is conducted by forming a bone tunnel with a drill guide that can be self-positioning. A self-positioning drill guide can allow centric drilling. A method can include determining a center point equidistant from the peripheral edges of the bone; and drilling the bone at the center point. A method can also include engaging a plurality of bone engaging structures (flexible wings or arms) of the drill guide with one or more peripheral edges of the bone; and drilling through a sleeve of the drill guide, through a center point, and into the bone to a desirable depth.

### BRIEF DESCRIPTION OF THE DRAWINGS

FIG. 1 illustrates an exemplary drill guide.
FIG. 2 illustrates an enlarged view of the drill guide of FIG. 1 (rotated 90 degrees).
FIGS. 3 and 4 illustrate steps of an exemplary method of reconstructive surgery with the drill guide of FIG. 1.
FIG. 5 illustrates another exemplary drill guide.
FIG. 6 illustrates an exemplary method of reconstructive surgery with the drill guide of FIG. 5.
FIG. 7 illustrates another exemplary drill guide.
FIG. 8 illustrates another exemplary drill guide.
FIG. 9 illustrates an exemplary method of reconstructive surgery with the drill guide of FIG. 8.
FIGS. 10-12 illustrate exemplary repairs with drill guides of the present disclosure.
FIG. 13 illustrates another exemplary drill guide.
FIG. 14 illustrates an exemplary method of reconstructive surgery with the drill guide of FIG. 13.
FIG. 15 illustrates another exemplary drill guide.
FIG. 16 illustrates another exemplary drill guide.
FIG. 17 illustrates an exemplary method of reconstructive surgery with the drill guide of FIGS. 15 or 16.
FIG. 18 illustrates another exemplary drill guide.
FIG. 19 illustrates another exemplary drill guide.
FIG. 20 illustrates another exemplary drill guide.
FIGS. 21-24 illustrate exemplary repairs with drill guides of the present disclosure.
FIG. 25 illustrates an exemplary method of reconstructive surgery with the drill guide of FIG. 7.

### DETAILED DESCRIPTION

The present disclosure provides methods and surgical constructs for fixation of tissue, for example, bone to bone.

A guide drill includes two or more flexible wings that reference two or more peripheral edges of one or more bones. In an embodiment, a cannulated drill sleeve of a drill guide is automatically positioned by a symmetrical spring system of the wings. A cannulated drill sleeve of a drill guide can be automatically centered by the symmetrical spring system of the wings. The material elongation of the wings works as a spring system which automatically positions (*e.g.*, centers and/or angles) one or more cannulated drill sleeves to one or more bones.

A drill guide can be a positioning drill guide. A drill guide can be a self-positioning drill guide. In an embodiment, a self-positioning drill guide is a center point drill guide configured to automatically center drill tunnels in bones by using peripheral edges to help precisely reference a center point. Various studies relating to AC (acromioclavicular) separation have shown that drilling tunnels in the center of the clavicle as well as the coracoid reduces the risk of fractures. The centering guide developed by the inventors helps in automatically determining the center of bones as well as providing more control during drilling. The guide could be applied also to other indications and procedures that require controlled drilling.

Open AC joint reconstructions typically require free hand drilling to be performed by surgeon (with a drill guide or even without a drill guide at all). The drill guide of the present disclosure can be clipped to (clamped onto) the bone and, therefore, always allows drilling at the desired location and or angle (angulation) relative to the bone edges. If drilling at center of bone is desired, the centric drilling occurs at a center point that is equidistant from the peripheral edges of the bone where the guide clips or clamps onto.

A drill guide can be an arthroscopic drill guide. A drill guide can position itself on a location on bone. A drill guide can be self-positioning and can further locate the center of a bone tunnel. The drill guide can include a shaft or sleeve with a plurality of flexible arms or flexible wings. The drill guide is positioned onto bone so that the flexible wings reference the peripheral edges of the bone. The drill guide is clipped or clamped onto/to the bone, thus always allowing precise drilling of the bone at the desired location and/or angle (the drill angle).

The surgical construct can be a "one piece" drill guide manufactured from plastics, metals, or similar materials, or combinations of such materials. The surgical construct is a simple instrument that allows precise, accurate, and convenient positioning on at least two sides/edges/surfaces of a bone to be drilled. The construct eliminates the free hand drilling in reconstruction surgeries such as open AC reconstruction. The construct of the present disclosure also allows drilling of tunnels at desired angle(s) and/or location(s) in two adjacent bones (such as through the clavicle, as well as the coracoid) to reduce the overall risk of fractures. The present disclosure also provides surgical techniques that allow for precise and automatic concentric drilling of bone tunnels.

Methods of endoscopic and/or arthroscopic surgeries are also disclosed. An exemplary method includes *inter alia* the steps of: (i) clamping a drill guide to a bone; and (ii) forming a bone tunnel into the bone and through the drill guide. The method further includes the steps of engaging a plurality of bone engaging structures (flexible wings or arms) of the drill guide with one or more bone sides/surfaces/edges of the bone; and drilling into the bone at the desired location and/or drill angle.

Another exemplary method includes *inter alia* the steps of: (i) determining a center point equidistant from peripheral edges of a bone; and (ii) forming a bone tunnel through the center point and into the bone. The method further includes the steps of engaging a plurality of bone engaging structures (flexible wings or arms) of a center point drill guide with one or more bone sides/surfaces/edges of the bone; and drilling into the bone and through the center point to a desirable depth. In an embodiment, drilling can occur from one surface of the bone to another surface of the bone and equidistant from peripheral edges of the bone, to form a through tunnel.

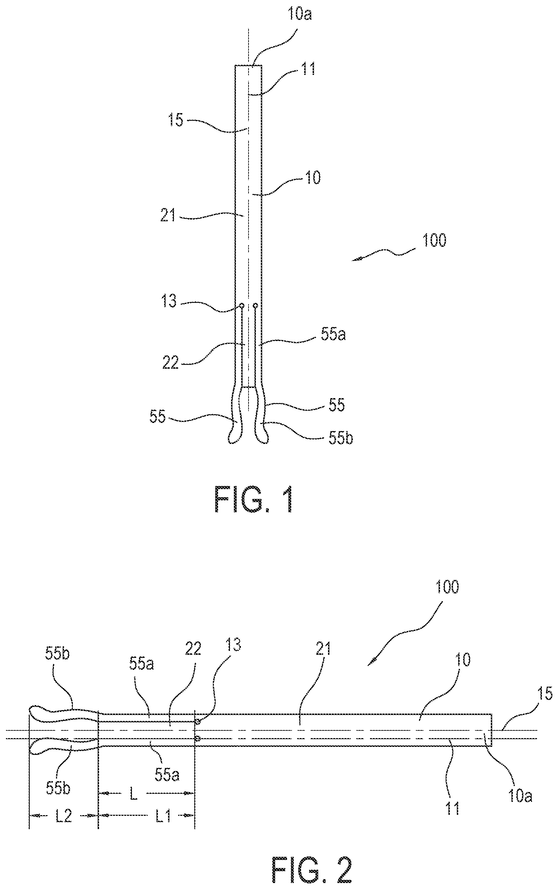

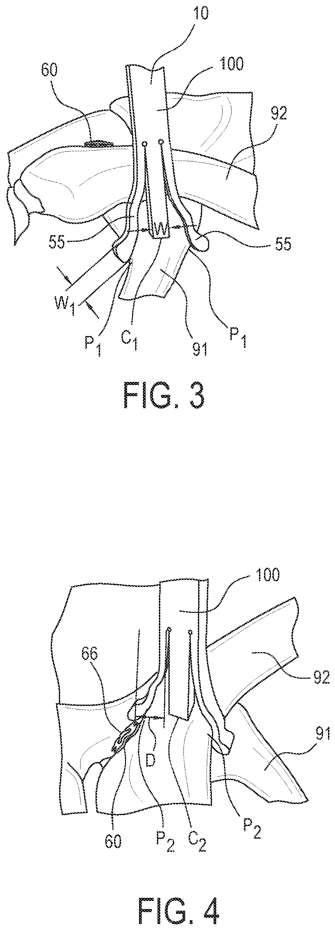

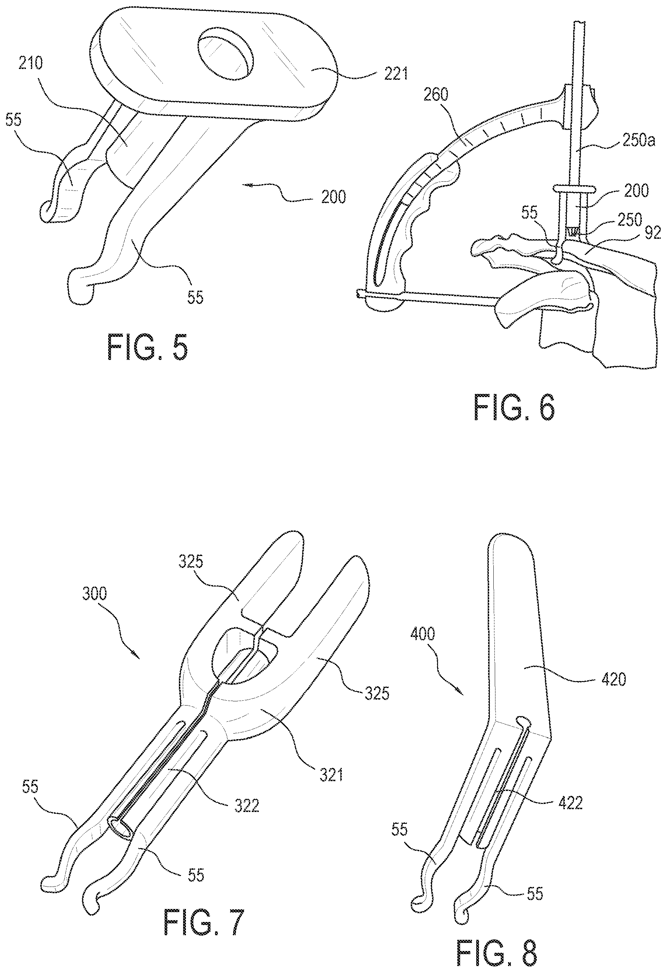

Referring now to the drawings, where like elements are designated by like reference numerals, FIGS. 1 and 2 illustrate exemplary drill guide 100 (self-positioning drill guide 100; center point drill guide 100; construct 100; surgical construct 100; centering bone drill guide 100). FIGS. 3 and 4 illustrate steps of drilling bone tunnels in different bones with exemplary drill guide 100 of FIG. 1. FIG. 5 illustrates exemplary drill guide 200 (self-positioning drill guide 200; center point drill guide 200; construct 200; surgical construct 200). FIG. 6 illustrates self-positioning drill guide 200 employed as an arthroscopic positioning guide in an arthroscopic repair such as an exemplary AC joint reconstruction (with self-positioning drill guide 200 engaging the clavicle).

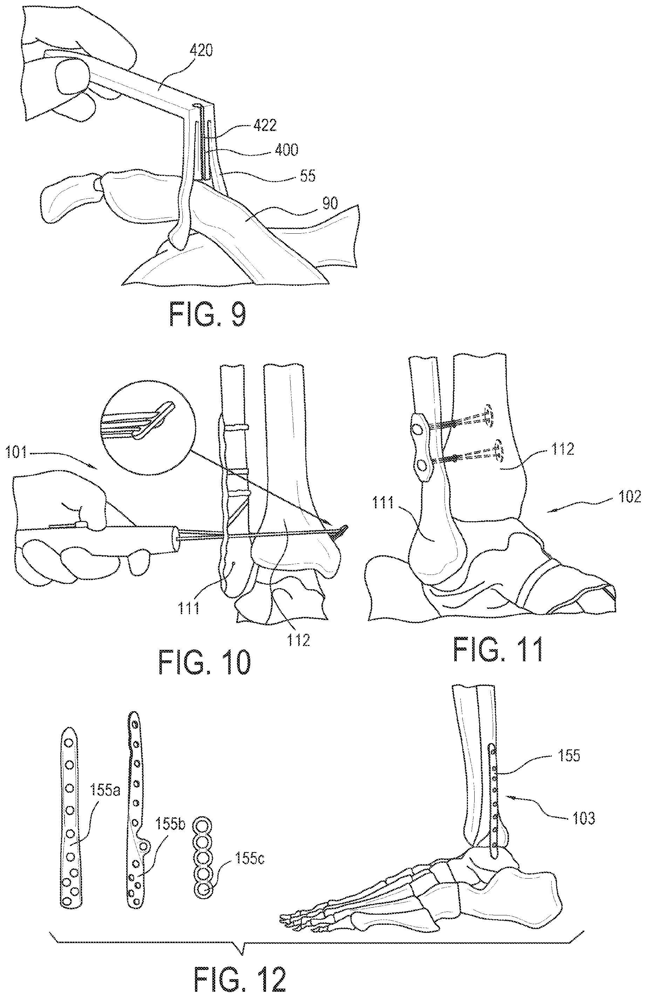

FIGS. 7 and 8 illustrate exemplary drill guide 300, 400 (self-positioning drill guide 300, 400; center point drill guide 300, 400; construct 300, 400; surgical construct 300, 400; centering bone drill guide 300, 400). FIG. 9 illustrates drill guide 400 of FIG. 8 employed in an exemplary AC joint reconstruction repair. FIGS. 10 and 11 illustrate drill guides 300, 400 employed as open positioning guides in exemplary syndesmosis repairs between fibula and tibia. FIG. 12 illustrates drill guides 300, 400 employed as open positioning guides in exemplary Tibia / Fibula / Ulna / Radius / Hand / Feet Fracture with Plate.

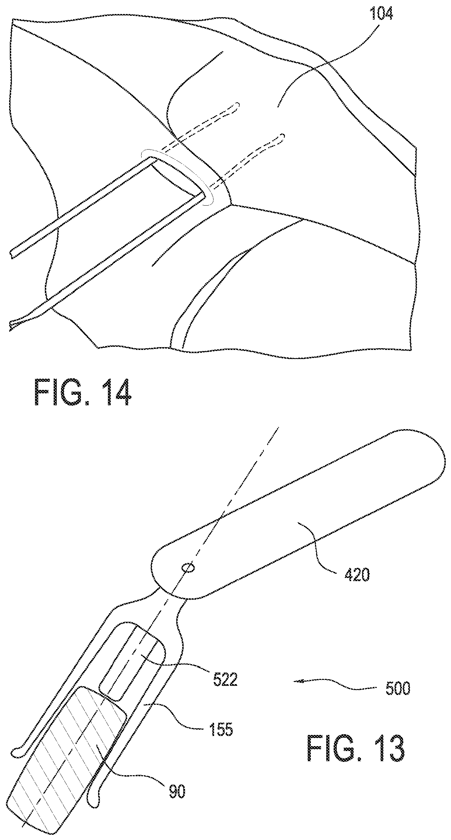

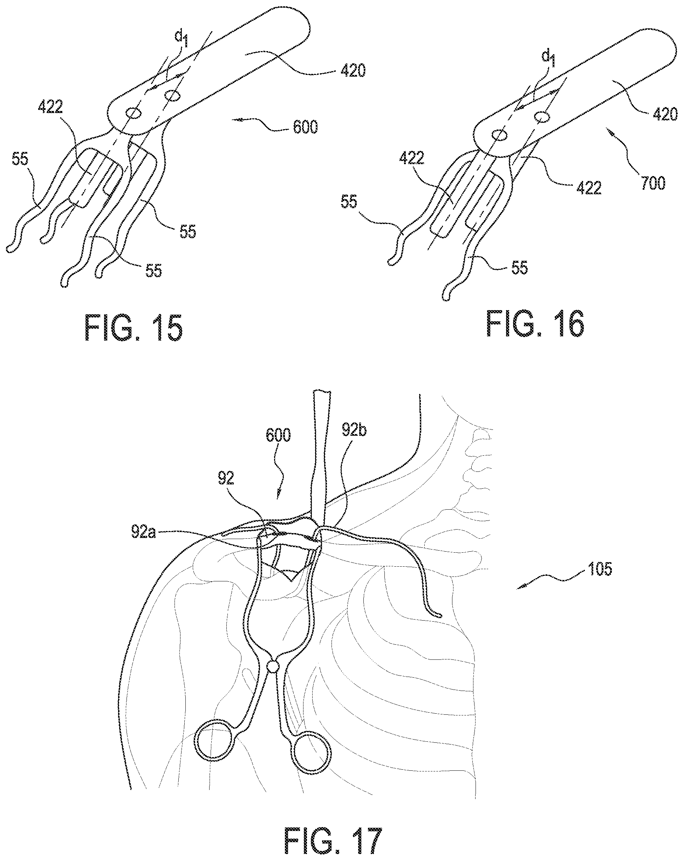

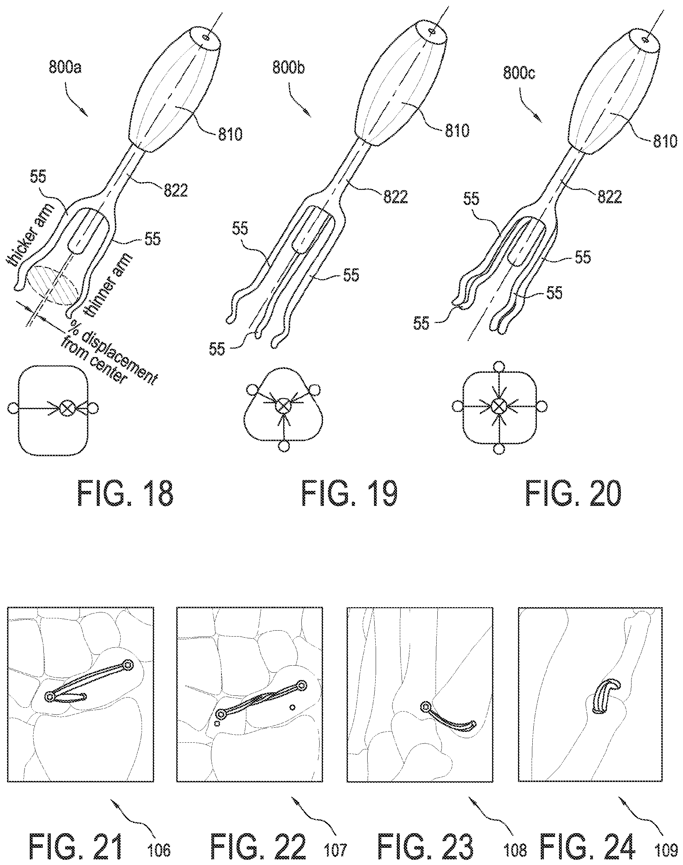

FIG. 13 illustrates exemplary drill guide 500 (self-positioning drill guide 500; center point drill guide 500; construct 500; surgical construct 500) which is a minimally invasive positioning guide for patella for an exemplary MPFL reconstruction (FIG. 14). FIGS. 15 and 16 illustrate drill guide 600, 700 (self-positioning drill guide 600, 700; center point drill guide 600, 700; construct 600, 700; surgical construct 600, 700; centering bone drill guide 600, 700) employed as clavicle positioning guide in exemplary clavicle Twin AC reconstruction (FIG. 17). FIGS. 18-20 illustrate drill guide 800a, 800b, 800c (self-positioning drill guide 800a, 800b, 800c; center point drill guide 800a, 800b, 800c; construct 800a, 800b, 800c; surgical construct 800a, 800b, 800c; centering bone drill guide 800a, 800b, 800c) employed in Internal Brace Positioning in Hand and Wrist applications (FIGS. 21-24).

Drill guide 100 of FIGS. 1 and 2 includes a cannulated shaft 10 (sleeve 10; tube 10) with a proximal end 11, a distal end 13 and a longitudinal axis 15. A handle (not shown) can be provided at the proximal end 11. Longitudinal axis 15 is preferably centrally located relative to the shaft 10. The shaft 10 is cannulated along its entire length to allow passage of one or more instruments, for example, drill pin, drill, or any cutting instrument (not shown) during a drilling procedure. The shaft 10 is provided with an open-ended bore 10a (for example, cylindrically shaped and longitudinally extending) provided along its length for receiving one or more instruments (for example, a drill) during use.

Shaft 10 includes an enlarged portion 21 and a narrower segment 22 extending along the longitudinal axis 15 and at the distal end 13 of shaft 10, and integral with the portion 21. Portion 21 has a diameter and/or width greater than the diameter and/or width of the narrower segment 22. Narrower segment 22 may have a circular or oval cross-section or any other cross section. For example, FIG. 1 illustrates narrower segment 22 having a square or rectangular cross-section and being flanked on two of its four sides by corresponding flexible wings. Both enlarged portion 21 and narrower segment 22 are cannulated to allow passage of additional instruments such as drills.

A plurality of flexible wings 55 (flexible arms 55; flexible engagement arms 55; flexible engagement segments 55) are provided at the distal end 13 of the shaft 10 and adjacent the narrower segment 22. Flexible arms 55 are designed to flex (flare out) when the drill guide is in contact with bone 91, 92 (as shown in FIGS. 3 and 4, for example) and to engage the bone (clamp onto the bone surfaces (sides or edges) and/or clip onto the peripheral edges). FIGS. 1 and 2 show the flexible arms 55 in the undeployed or unflexed position, *i.e.,* when the arms 55 do not extend away from shaft 10. FIGS. 3 and 4 show the flexible arms in the deployed or flexed position, *i.e.,* when the flexible arms extend away from the shaft 10 (flare out) for a distance "D" (FIG. 4) to clamp onto peripheral edges P1 of the coracoid 91 (FIG. 3) and securely attach to coracoid 91, and/or to clamp onto peripheral edges P2 of the clavicle 92 (FIG. 4) and securely attach to clavicle 92.

Flexible arms 55 can be resiliently flexible and formed of materials that allow such resilient flexibility. Flexible arms 55 can be formed of a material similar to or different from that of shaft 10. In an embodiment, flexible arms 55 can be made of metal, polymer, plastic, fiberglass, or other material or mixture of materials that provide suitable flexibility. Suitable polymeric materials include polycarbonate, polyethylene, polyurethane, polyolefin, and other materials which may be used in fabrication of part or all of shaft 10 and flexible arms 55. In an embodiment, flexible arms 55 are formed of plastic materials and the guide 100 is formed by molding, for example, insert molding or milling or other known methods in the art. In another embodiment, flexible arms 55 are formed of a material with memory properties which allows the arms to bend and to flex yet return to their initial position (undeployed position) once the pushing force is removed. The flexible arms can be attached to the shaft 10 by gluing, bonding, fusing, melting, heating, or by any other similar methods known in the art to bring two or more pieces of plastic, metals or materials together. The flexible arms may be formed of Nitinol.

FIGS. 1 and 2 illustrate two exemplary flexible arms 55 integrally formed with shaft 10. However, the disclosure is not limited to this exemplary-only embodiment and contemplates drill guides with any number of flexible arms, as desired and depending on the configuration of the bones or segments, or bone systems, to be drilled.

In the embodiment shown in FIGS. 1 and 2, flexible arms 55 are symmetrically located relative to the axis 15 and to the narrower segment 22 of drill guide 100. In an embodiment, flexible arms 55 are fixedly and securely attached to the portion 21 of shaft 10, and symmetrically positioned relative to a plan passing through the center line of the shaft 10 and along longitudinal axis 15. In an embodiment, flexible arms 55 are fixedly and securely attached to the portion 21 of shaft 10, and symmetrically positioned relative to the narrower segment 22, *i.e.,* disposed on two opposite lateral sides of segment 22.

Each arm 55 is provided with a relatively straight and substantially linear portion 55a and with a curved or bent portion or segment 55b (bowed portion or segment 55b; arched portion or segment 55b). As shown more clearly in FIG. 2, the length L1 of the linear portion 55a is about similar to the length L of narrower segment 22. The length L2 of the curved portion 55b is about similar to length L1. The width W1 (FIG. 3) of the flexible arms 55 is about similar to width W (FIG. 3) of the narrower portion 22. Curved portions 55b extend past a most distal end of shaft 10 (fully cannulated sleeve 10) by distance L2.

Curved portion 55b has an inner surface that is configured to resemble the approximal average surface of peripheral edges P1, P2 of bone 91, 92 (FIGS. 3 and 4) to be drilled, *e.g*., coracoid 91 and clavicle 92. When the drill guide is clipped or clamped onto bone 91, 92, each of the flexible arms 55 flexes relative to the longitudinal axis 15 and securely engages each of the peripheral edges of bone 91, 92. The flexible arms 55 reference peripheral edges/surfaces of the bone and the cannulated shaft 10 (sleeve 10) is automatically centered on bone 91, 92 by the symmetrical spring system of the flexible arms 55 (wings 55). Once the guide 100 is clipped or clamped onto the bone 91, 92 (attached, clasped, or fastened to the bone 91, 92), the surgeon could drill a bone tunnel into the bone and through the bone (for example, at the center of the bone), eliminating therefore the necessity of free hand drilling. When the drill guide is clipped or clamped onto the bone, the direction of the drill bit coincides with the center of the bone and, thus, the drilled bone tunnel is located in the middle of the bone. The guide centers the drill through the sleeve of the guide. The independent flexible arms/wings are equally tensioned and they reference where the cannulation of the guide goes (the center cannulation is automatically centered by the equally-tensioned flexible arms).

Reference is now made to FIGS. 3 and 4, which illustrate steps of a method of AC reconstruction with the drill guide 100 of FIG. 1. FIG. 3 depicts drill guide 100 securely engaging first bone 91 (coracoid 91; coracoid process 91) to form (drill) a first bone tunnel 91a. Drill guide 100 is clipped/clamped onto coracoid process 91 to allow drilling of coracoid tunnel 91a in the center of coracoid 91. FIG. 4 depicts drill guide 100 securely engaging second bone 92 (clavicle 92) to form (drill) a second bone tunnel 92a. Drill guide 100 is clipped/clamped onto clavicle 92 to allow drilling of clavicle tunnel 92a in the center of clavicle 92. Drill guide 100 can drill both tunnels 91a, 92a in the center C1, C2 of each bone 91, 92 reducing, therefore, the risk of fractures in AC reconstruction and providing increased control during the drilling of the bone tunnels. The formation of the first tunnel 91a may occur before or after the formation of the second tunnel 92a, and may be conducted with a same or different drill guide. In certain embodiments, and depending on the number of flexible arms 55, the formation (drilling) of the first tunnel can occur simultaneously with the formation of the second tunnel (*i.e.,* with a same drill guide that allows a same drill bit to pass through the center of the clavicle and the center of the coracoid, without removing the guide from the arthroscopic site; or with the same drill guide having two or more cannulated sleeves to allow two or more cutting instruments (drills) to pass therethrough).

Clamping/clipping/securing of the drill guide 100 onto peripheral edges P1, P2 of each bone 91, 92 allows determining a reference drill point or location, for example, a center point C1, C2 equidistant from opposed peripheral edges P1, P2 of bone 91, 92. Narrower section 22 of shaft 10 is located over and in contact with the center point C1 (FIG. 3) and C2 (FIG. 4) to allow formation of tunnels 91a, 92a (not shown) at the center point C1, C2 and through the bone 91, 92. Clamping of the drill guide to the bone occurs by clamping the flexible wings 55 to peripheral edges of the bone so that curved sections 55b of the flexible arms follow the contour of each of the bone edges and securely engage the bone. The material elongation of the wings 55 acts as a spring system that automatically positions and/or angles the cannulated drill sleeve to the bone. The automatic position may occur at the center of the bone, but the positioning may also occur at any other desired location on the bone.

FIG. 4 also illustrates a fixation device 60 in the form of a button 60 (which may be metallic or non-metallic) that allows flexible strands such as sutures 66 and/or tapes 66 and optionally a graft to pass through the tunnels formed in both the clavicle and the coracoid in an AC joint repair. In an exemplary embodiment and as shown in FIG. 4, the fixation device 60 is a dog bone-shaped button and at least one suture and/or tape 66 is used to secure the buttons during an AC joint repair. Details of fixation device 60 in the form of a dog bone-shaped button as part of an AC joint fixation system are set forth in US 9,421,007 issued Aug. 23, 2016 entitled "Acromioclavicular Joint Fixation Using Suture Button Construct with Dog Bone-Shaped Button". As detailed in US 9,421,007, the fixation device 60 may be part of a system with two buttons 60 joined together by flexible strands 66 in the form of suture or tape. The suture may be made of any known suture construct, such as multifilament, braided, knitted, woven suture, or including fibers of ultrahigh molecular weight polyethylene (UHMWPE) or the FiberWire^{®} suture (disclosed in US 6,716,234). The tape may be formed of suture tape, for example, Arthrex FiberTape^{®}, which is a high strength suture tape that is braided and rectangular-like in cross section and as disclosed in US 7,892,256.

FIG. 5 illustrates drill guide 200 (self-positioning drill guide 200; center point drill guide 200; construct 200; surgical construct 200) according to another exemplary embodiment. Drill guide 200 of FIG. 5 is about similar to drill guide 100 of FIG. 1 in that drill guide 200 is also provided with a plurality of flexible wings 55 (flexible arms 55; flexible engagement arms 55; flexible engagement segments 55) provided at the distal end of the instrument and in the proximity of a cannulated segment 210 or cannulated sleeve 210 (corresponding to shaft 10 of drill guide 100). Flexible arms 55 are designed to flex and flare out when the drill guide 200 is in contact with bone 92 (as shown in FIG. 6, for example) and engage peripheral edges of the bone 92 (clavicle 92).

Drill guide 200 differs from drill guide 100, however, in that drill guide 200 is provided with a wider portion 221 that (together with sleeve 210) define a passage for drill bit 250 (FIG. 6) to pass therethrough.

FIG. 6 illustrates drill guide 200 clipped/clamped on clavicle 92 with flexible arms 55 engaging two peripheral edges P1 of the clavicle 92 to allow drilling of bone tunnel 92a through the clavicle 92. FIG. 6 illustrates guide 200 employed as an arthroscopic positioning guide 200 positioned on clavicle 92 for an exemplary AC joint reconstruction. The drill guide 200 can be applied on, and employed with, exemplary arthroscopic Drill Guide 260. Drill guide 200 is clipped on clavicle 92 (with flexible arms 55 engaging peripheral edges of clavicle 92) and centers Drill Guide 260 with drill 250 (drill bit 250; cutting instrument 250) on clavicle 92. Drill guide 200 can be movable on the drill sleeve 250a of arthroscopic drill guide 260 (*i.e.,* it can slide up and down axially along the drill sleeve 250a).

FIGS. 7 and 8 illustrate drill guide 300, 400 (self-positioning drill guide 300, 400; center point drill guide 300, 400; construct 300, 400; surgical construct 300, 400; centering bone drill guide 300, 400) according to additional exemplary embodiments. Drill guide 300 of FIG. 7 is about similar to drill guide 100 of FIG. 1 in that drill guide 300 is also provided with a plurality of flexible wings 55 (flexible arms 55; flexible engagement arms 55; flexible engagement segments 55) provided at the distal end of the instrument and in the proximity of a cannulated segment 322 or sleeve 322 (corresponding to narrower segment 22 of drill guide 100). Flexible arms 55 are designed to flex and flare out when the drill guide 300 is in contact with bone and engages peripheral edges of the bone. Drill guide 300 differs from drill guide 100, however, in that drill guide 300 is provided with a wider portion 321 in the shape of a handle with two arms 325 that define a passage for a specific instrument handle to pass therethrough.

Drill guide 400 of FIG. 8 is about similar to drill guide 300 of FIG. 7 in that drill guide 400 is also provided with a plurality of flexible wings 55 (flexible arms 55; flexible engagement arms 55; flexible engagement segments 55) provided at the distal end of the instrument and in the proximity of a cannulated segment 422 or sleeve 422 (corresponding to narrower segment 22 of drill guide 100). Flexible arms 55 are designed to flex and flare out when the drill guide 400 is in contact with bone 90 and engages peripheral edges of the bone 90 (as shown in FIG. 9). Bone 90 can be the clavicle. Drill guide 400 differs from drill guide 300, however, in that drill guide 400 is provided with a handle/tab 420 *in lieu* of the wider portion 321 of guide 300. FIG. 9 illustrates guide 400 used as an open positioning guide for exemplary Clavicle / Coracoid / Acromion for AC Joint reconstruction. Guide 400 is applied on Clavicle, Coracoid and/or Acromion and can be clipped on any of these bones. The guide centers the drill through the cannulated sleeve 422.

FIGS. 10-12 illustrate various applications and/or surgical repairs 101, 102, 103 with drill guides of the present disclosure. Repairs 101 and 102 (conducted with any open positioning guide of the present disclosure) are exemplary syndesmosis repairs 101, 102 between fibula 111 and tibia 112. The guide is clipped on fibula 111. The surgeon drills through the centered drill guide sleeve and tibia 112. After drilling, a Tightrope^{®} system can be employed, such as Arthrex Syndesmosis TightRope^{®} XP implant system.

FIG. 12 illustrates repair 103 with drill guides of the present disclosure which are employed as open positioning guides for any of Tibia / Fibula / Ulna / Radius / Hand / Feet Fracture with plate. The guide is clipped on the specific bone with a bone plate such as plate 155, 155a, 155b, 155c (as also shown in FIG. 12). The drill is centered on bone through the drill guide sleeve. The drill guide positions the plate which is centered on bone with positioned drill holes. The repair also applies to long straight plates.

FIG. 13 illustrates yet another embodiment of a self-positioning guide of the present disclosure. Drill guide 500 (self-positioning drill guide 500; construct 500; surgical construct 500; centering bone drill guide 500) is a minimally invasive positioning guide employed on patella 93 for an exemplary MPFL reconstruction repair 104 (FIG. 14).

FIGS. 15 and 16 illustrate exemplary drill guide 600, 700 (self-positioning drill guide 600, 700; center point drill guide 600, 700; construct 600, 700; surgical construct 600, 700; centering bone drill guide 600, 700). FIG. 17 illustrates a view of human shoulder undergoing AC joint reconstruction with any of guides 600, 700.

Drill guides 600, 700 are about similar to guide 400 of FIG. 8 but differ in that they include two or more sleeves 422 for allowing two or more drill bits. Guide 600 includes four exemplary flexible arms 55 and two exemplary sleeves 422; guide 700 includes two exemplary flexible arms 55 and two exemplary sleeves 422. Sleeves 422 may be about parallel to each other and spaced apart from each other by a distance "d1." In an embodiment, sleeves 422 can extend about perpendicular to handle 420. In other embodiments, sleeves 422 can be non-parallel to each other and/or can extend in directions other than perpendicular to handle 420, depending on the bone surface and configuration of the bone or bones to be drilled. The two bone tunnels formed with each of guides 600, 700 can be formed simultaneously or sequentially, as desired.

FIG. 17 illustrates a clavicle Twin AC Joint reconstruction 105. The guide 600 or 700 engages clavicle 92 and is clipped on the clavicle, centering drilling for both drill holes 92a, 92b on the clavicle. The drill guide is provided with two or more sleeves spaced apart by a fixed distance, and with two or more flexible arms (for example, four flexible arms 55 as in exemplary guide 600).

FIGS. 18-20 illustrate exemplary drill guides 800a, 800b, 800c which are about similar to the guides described above in that guides 800a, 800b, 800c are also self-positioning drill guides that can be centering drill guides (center point drill guides). Drill guides 800a, 800b, 800c are about similar to each other but differ in the number of flexible arms 55. Guide 800a is provided with two exemplary flexible arms 55; guide 800b is provided with three exemplary flexible arms 55; guide 800c is provided with four exemplary flexible arms 55. Guides 800a, 800b, 800c each can have a handle 810 extending along cannulated sleeve 822.

Particular applications of guides 800a, 800b, 800c are Internal Brace Positioning in Hand and Wrist repairs, where the guide can clip on lunate and/or scaphoid and allow drilling at various anatomical positions, as shown in repairs 106-109 of FIGS. 21-24 (dorsal scapholunate reconstruction 106 of FIG. 21; interosseous scapholunate reconstruction 107 of FIG. 22; InternalBrace ligament augmentation repair with APL suspensionplasty 108 of FIG. 23; and thumb UCL repair 109 of FIG. 24).

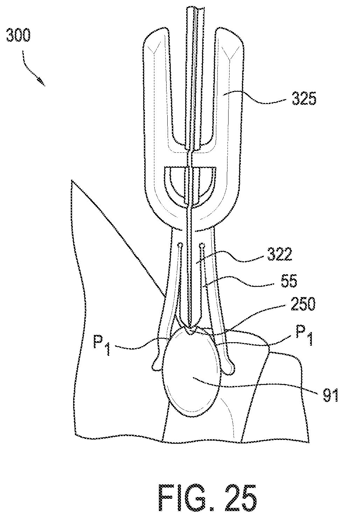

FIG. 25 illustrates exemplary drill guide 300 clipped/clamped on coracoid 91 with flexible arms 55 engaging two peripheral edges P1 of the coracoid 91 to allow centric drilling of bone tunnel 91a through the coracoid 91. The formation of clavicle tunnel 92a can occur before or after the formation of the coracoid tunnel 91a and can be conducted with a same or different drill guide.

Although drill guides 100, 200, 300, 400, 500, 600, 700, 800a, 800b, 800c have been described above with reference to only two, three, or four exemplary flexible arms or wings 55, 155, the disclosure is not limited to these exemplary-only embodiments, and it has applicability to surgical drill guides with any number of flexible arms or wings having similar or different configurations. For example, a surgical drill guide of the present disclosure employed in AC reconstruction can be provided with three flexible wings, with one of the three flexible wings referencing the posterior side of the clavicle while the other two wings referencing the coracoid (for example, two different sides/surfaces/peripheral edges of the coracoid). In this manner, the surgeon can directly drill the bones (for example, the center of both bones) in a single step, with minimal damage to the bones and increased accuracy with respect to the center drilling of the bones. In this embodiment, formation of centric clavicle and coracoid tunnels may be conducted simultaneously, and with a same instrument, and without removing the drill bit from the surgical site.

Any of drill guides 100, 200, 300, 400, 500, 600, 700, 800a, 800b, 800c described above can be included in a surgical kit or system to simplify the surgeon's task of selecting a specific instrument and aid in the overall surgical procedure. A surgical kit for an orthopedic surgical repair may include one or more drill guides 100, 200, 300, 400, 500, 600, 700, 800a, 800b, 800c and an obturator sized for use with the drill guide 100, 200, 300, 400, 500, 600, 700, 800a, 800b, 800c. The surgical kit can also include awls or equivalent devices, as well as drills, pins, cutting instruments or bone-penetrating devices. The surgical kit can also include additional fixation devices such as fixation devices 60 with flexible strands 66 detailed above and/or suture anchors to be employed in conjunction with the bone tunnels, bores or holes formed by the drill guide 100, 200, 300, 400, 500, 600, 700, 800a, 800b, 800c.

In an exemplary embodiment, the suture anchor may be a soft anchor (soft suture anchor) provided with a soft anchor sleeve (sheath, tubular member) with two open ends and one or more flexible shuttling strands extending through the soft anchor sleeve (sheath). The at least two flexible strands may extend through the sleeve in similar or different directions and/or orientations and/or locations. The flexible sleeve with the one or more shuttling strands is secured into or onto bone, and the strands allow passing of additional flexible strands such as tapes to pass over soft tissue and be secured into bone to approximate soft tissue to bone. Details of an exemplary soft suture anchor with a soft anchor sleeve (sheath or tubular member) and flexible shuttling strands are set forth, for example, in US Application Serial No. 15/998,516 entitled "Methods of Tissue Repairs" filed on Aug. 16, 2018.

Drill guides 100, 200, 300, 400, 500, 600, 700, 800a, 800b, 800c may be reusable or disposable (single use) devices.

Drill guides 100, 200, 300, 400, 500, 600, 700, 800a, 800b, 800c detailed above have applicability to various open or arthroscopic procedures including procedures for re-approximating bone to bone or soft tissue to bone, for example, shoulder rotator cuff repairs, capsulolabral reconstruction, SLAP repairs, as well as ankle, knee, elbow, hand, wrist, or foot repairs. Drill guides 100, 200, 300, 400, 500, 600, 700, 800a, 800b, 800c have particular application to AC joint repairs wherein the tunnels drilled through the bones (*i.e.,* the clavicle and coracoid) pose potential fractures for the clavicle and coracoid. Disruption of the coracoclavicular ligaments requires the native ligaments to heal properly or secondary fixation in the clavicle and the subcoracoid, to restore the stability of the joint. With drill guide 100, 200, 300, 400, 500, 600, 700, 800a, 800b, 800c, secondary fixation in AC join repair is greatly improved.

A drill guide 100, 200, 300, 400, 500, 600, 700, 800a, 800b, 800c has a plurality of flexible wings 55, 155 that reference peripheral edges P1, P2 of bone 90, 91, 92, 93. A cannulated centered drill sleeve 10 is automatically positioned (for example, centered) at a desired location by the symmetrical spring system of the plurality of flexible wings 55, 155.

Guide 100, 200, 300, 400, 500, 600, 700, 800a, 800b, 800c is securely clipped to (or clamped onto) the bone 90, 91, 92, 93 (without moving) and thus always allows precise drilling at the desired location and/or angle. Guide 100, 200, 300, 400, 500, 600, 700, 800a, 800b, 800c has particular application to open AC reconstruction, wherein drilling of tunnels in the center of the clavicle as well as the center of the coracoid reduces the risk of fractures. By replacing free hand drilling in open AC reconstruction, the technique allows for precise and automatic concentric drilling of bone tunnels.

A drill guide 100, 200, 300, 400, 500, 600, 700, 800a, 800b, 800c can automatically center drill tunnels 91a, 92a in bones 90, 91, 92, 93 by using peripheral edges P1, P2 that precisely reference center point C1, C2. The guide helps to automatically determine the center C1, C2 of bones 90, 91, 92, 93 as well as to provide increased control during drilling.

A method of forming a bone hole or tunnel 91a, 92a into bone 90, 91, 92, 93 comprises *inter alia* the steps of: (i) selecting a site on bone 90, 91, 92, 93 for forming a bone hole or tunnel 91a, 92a; (ii) self-positioning a drill guide 100, 200, 300, 400, 500, 600, 700, 800a, 800b, 800c at the site, wherein the drill guide has a shaft 10 with a proximal end 11, a distal end 13, a longitudinal axis 15, and a cannulation along the longitudinal axis 15, and a plurality of flexible arms 55, 155 located at the distal end 13 of the shaft 10; and (iii) forming a bone hole or tunnel 91a, 92a in bone 90, 91, 92, 93 and through drill guide 100, 200, 300, 400, 500, 600, 700, 800a, 800b, 800c. The method may further include the steps of: referencing at least two peripheral edges P1, P2 of the bone 90, 91, 92, 93 with the plurality of flexible arms 55, 155; determining a center C1, C2 of the bone 90, 91, 92, 93; and drilling the bone tunnel 91a, 92a through the drill guide, in the center C1, C2 of the bone 90, 91, 92, 93, and at a mid-distance between the at least two peripheral edges P1, P2. The bone tunnels may be formed simultaneously or sequentially.

A method of AC joint reconstruction comprises *inter alia* the steps of: (i) clamping a drill guide 100, 200, 300, 400, 500, 600, 700, 800a, 800b, 800c onto peripheral edges P1 of a first bone 91, the drill guide 100, 200, 300, 400, 500, 600, 700, 800a, 800b, 800c comprising a cannulated sleeve 10 and a plurality of flexible arms 55, 155 extending away from a distal end 13 of the cannulated sleeve 10, at least two of the plurality of flexible arms 55 being positioned symmetrically relative to a longitudinal axis 15 of the cannulated sleeve 10 equidistant to the longitudinal axis 15; and (ii) forming a first bone tunnel 91a through the first bone 91. The method further comprises the steps of: (iii) clamping the drill guide 100, 200 onto peripheral edges P2 of a second bone 92; and (iv) forming a second bone tunnel 92a through the second bone. The first bone 91 may be clavicle and the second bone 92 may be coracoid. The first bone 91 may be coracoid and the second bone 92 may be clavicle. The method may further include the steps of forming a first bone tunnel 91a in a center C1 of the first bone 91; and forming a second bone tunnel 92a and in a center C2 of the second bone 92. The method further comprises the steps of: placing a curved portion 55b of each of the at least two of the plurality of flexible arms 55 in contact with a respective bone surface of the bone 91, 92, so that each of the curved portion 55b follows a contour of the respective bone surface at peripheral edges P1, P2; referencing at least two bone edges P1, P2 with the at least two of the plurality of flexible arms 55; and determining a point C1, C2 equidistant from the at least two bone edges P1, P2 of each of the two bones.

Methods of surgeries are also disclosed. In an embodiment, a method of positioning and drilling a bone tunnel 91a, 92a in a bone 90, 91, 92 is conducted by forming a bone tunnel 91a, 92a with a drill guide 100, 200, 300, 400, 500, 600, 700, 800a, 800b, 800c that allows self-positioning and drilling at a desired location and/or angle on and relative to the bone (for example, the center of bone). A method of positioning and drilling a bone tunnel in a bone can include determining a center point C1, C2 equidistant from the peripheral edges P1, P2 of the bone 90, 91, 92; and drilling the bone 90, 91, 92 at the center point C1, C2. A method of positioning and drilling a bone tunnel in a bone can also include engaging a plurality of bone engaging structures 55, 155 (flexible wings or arms 55, 155) of drill guide 100, 200, 300, 400, 500, 600, 700, 800a, 800b, 800c with one or more peripheral edges P1, P2 of the bone 90, 91, 92, 93; and drilling through the center point C1, C2 and into the bone 90, 91, 92, 93 to a desirable depth.

A drill guide 100, 200, 300, 400, 500, 600, 700, 800a, 800b, 800c comprises at least one cannulated shaft or sleeve 10, 21, 22, 210, 322, 422, 522, 822 with a longitudinal axis 15, a proximal end and a distal end; and a plurality of flexible arms 55, 155 located at the distal end of the at least one cannulated shaft, wherein at least one of the plurality of flexible arms 55, 155 is adapted to reference a peripheral edge P1, P2 of a bone 90, 91, 92, 93 to be drilled. The drill guide 100, 200, 300, 400, 500, 600, 700, 800a, 800b, 800c can be self-positioning. The drill guide 100, 200, 300, 400, 500, 600, 700, 800a, 800b, 800c can be self-centering. The drill guide 100, 200, 300, 400, 500, 600, 700, 800a, 800b, 800c can be a single-use device or reusable.

A surgical kit comprises (i) a drill guide 100, 200, 300, 400, 500, 600, 700, 800a, 800b, 800c including a cannulated shaft or sleeve 10, 21, 22, 210, 322, 422, 522, 822 with a longitudinal axis, a proximal end and a distal end; a plurality of flexible arms or wings 55, 155 located at the distal end of the cannulated shaft, wherein the plurality of flexible arms or wings 55, 155 reference peripheral edges P1, P2 of one or more bones 90, 91, 92, 93 to be drilled; and (ii) a cutting instrument 250. The surgical kit can further comprise an obturator; and/or a fixation device 60. The fixation device 60 can be a metallic button; or a soft suture anchor comprising a flexible tubular sleeve or sheath and a plurality of flexible strands extending through a passage of the flexible tubular sleeve or sheath.

A method of forming a bone tunnel or hole 91a, 92a in a bone 90, 91, 92, 93 comprises *inter alia* the steps of: (i) determining a reference drill point of a bone tunnel or hole to be formed in bone by referencing opposite peripheral edges of the bone 90, 91, 92, 93; and (ii) drilling the bone tunnel or hole at the reference drill point and through the bone. The reference drill point can be the center point of the bone 90, 91, 92, 93. The method can further include the steps of: (iii) clamping a drill guide 100, 200, 300, 400, 500, 600, 700, 800a, 800b, 800c comprising at least one cannulated shaft or sleeve 10, 21, 22, 210, 322, 422, 522, 822 with a longitudinal axis, a proximal end and a distal end; and a plurality of flexible arms or wings 55, 155 located at the distal end of the cannulated shaft or sleeve 10, 21, 22, 210, 322, 422, 522, 822, wherein the plurality of flexible arms or wings 55, 155 reference opposite peripheral edges of the bone; and (iv) cutting through the drill guide and through the reference drill point. The bone 90, 91, 92, 93 can be the clavicle or the coracoid. The bone 90, 91, 92, 93 can be part of hand, foot, shoulder, elbow, ankle, wrist or arm. The bone 90, 91, 92, 93 can be part of an anatomical joint. The plurality of flexible arms or wings 55, 155 can be formed of a resilient plastic and can be integral with the cylindrical cannulated shaft.

## Claims

1. A drill guide (100) comprising:
at least one cannulated shaft (10) with a longitudinal axis, a proximal end and a distal end, the cannulated shaft (10) including a cylindrically shaped open-ended bore (10a), the open-ended bore (10a) extending longitudinally along the cannulated shaft (10); and
a plurality of resiliently flexible arms located at the distal end of the at least one cannulated shaft (10), wherein at least two of the plurality of resiliently flexible arms are adapted to reference opposite peripheral edges of the bone to be drilled, wherein the plurality of flexible arms (55, 155) are formed of a resilient plastic or metal and are integrally formed with the cannulated shaft (10) so that the resiliently flexible arms and the cannulated shaft (10) form a one-piece construct manufactured from resilient plastic or metal,
**characterised in that** the drill guide (100) is configured to automatically determine a center point (C1, C2) of a bone (90, 91, 92, 93) to be drilled, the center point (Cl, C2) being equidistant from peripheral edges (P1, P2) of the bone.

2. The drill guide (100) of claim 1, wherein the drill guide (100) is self-positioning and may further be self-centering.

3. The drill guide (100) of claim 1, wherein the bone is clavicle or coracoid or fibula or patella.

4. The drill guide (100) of claim 1, wherein the plurality of flexible arms (55, 155) consists of three flexible arms (55, 155) that reference peripheral edges of two bones.

5. The drill guide (100) of claim 4, wherein the two bones are clavicle and coracoid.

6. The drill guide (100) of claim 4, wherein one of the three flexible arms (55, 155) references one side of the bone, and the other two of the three flexible arms reference another bone.

7. The drill guide (100) of claim 1, wherein the at least one of the plurality of flexible arms (55, 155) comprises a linear segment and a curved or bowed segment, the curved or bowed segment extending past a most distal end of the cannulated shaft (10).

8. The drill guide (100) of claim 1, wherein the plurality of flexible arms (55, 155) is symmetrically located relative to the longitudinal axis.

9. The drill guide (100) of claim 1, comprising two cannulated shafts (10) to drill two bone tunnels or holes in the bone.

## Patentansprüche

1. Bohrerführung
(100), umfassend:
mindestens einen kanülierten Schaft (10) mit einer Längsachse, einem proximalen Ende und einem distalen Ende , wobei der kanülierte Schaft (10) eine zylindrisch geformte Bohrung (10a) mit offenem Ende umfasst, wobei sich die Bohrung (10a) mit offenem Ende in Längsrichtung entlang des kanülierten Schafts (10) erstreckt; und
eine Vielzahl von elastisch flexiblen Armen, die sich am distalen Ende des mindestens einen kanülierten Schafts (10) befinden, wobei mindestens zwei der Vielzahl von elastisch flexiblen Armen angepasst sind, auf gegenüberliegende Umfangskanten des zu bohrenden Knochens Bezug zu nehmen, wobei die Vielzahl von flexiblen Armen (55, 155) aus einem elastischen Kunststoff oder Metall bestehen und einstückig mit dem kanülierten Schaft (10) ausgebildet sind, sodass die elastisch flexiblen Arme und der kanülierte Schaft (10) ein einstückiges, aus elastischem Kunststoff oder Metall hergestelltes Teil bilden.
**dadurch gekennzeichnet, dass** die Bohrführung (100) konfiguriert ist, um automatisch einen Mittelpunkt (Cl, C2) eines zu bohrenden Knochens (90, 91, 92, 93) zu bestimmen, wobei der Mittelpunkt (Cl, C2) von Umfangskanten (P1, P2) des Knochens äquidistant ist.

2. Bohrführung (100) nach Anspruch 1, wobei die Bohrführung (100) selbstpositionierend ist und ferner selbstzentrierend sein kann.

3. Bohrführung (100) nach Anspruch 1, wobei der Knochen Clavicula oder Coracoid oder Fibula oder Patella ist.

4. Bohrführung (100) nach Anspruch 1, wobei die Vielzahl von flexiblen Armen (55, 155) aus drei flexiblen Armen (55, 155) besteht, die auf Umfangskanten von zwei Knochen Bezug nehmen.

5. Bohrführung (100) nach Anspruch 4, wobei die beiden Knochen Clavicula und Coracoid sind.

6. Bohrführung (100) nach Anspruch 4, wobei einer der drei flexiblen Arme (55, 155) auf eine Seite des Knochens Bezug nimmt und die anderen beiden der drei flexiblen Arme auf einen anderen Knochen Bezug nehmen.

7. Bohrführung (100) nach Anspruch 1, wobei mindestens einer der Vielzahl von flexiblen Armen (55, 155) ein lineares Segment und ein gekrümmtes oder gebogenes Segment umfasst, wobei sich das gekrümmte oder gebogene Segment über ein am weitesten distales Ende des kanülierten Schafts (10) hinaus erstreckt.

8. Bohrführung (100) nach Anspruch 1, wobei die Vielzahl von flexiblen Armen (55, 155) symmetrisch relativ zu der Längsachse angeordnet ist.

9. Bohrführung (100) nach Anspruch 1, die zwei kanülierte Schäfte (10) zum Bohren von zwei Knochentunneln oderlöchern in dem Knochen umfasst.

## Revendications

1. Guide de forage (100) comprenant :
au moins une tige tubulaire (10) ayant un axe longitudinal, une extrémité proximale et une extrémité distale, la tige tubulaire (10) comportant un alésage à extrémité ouverte (10a) de forme cylindrique, l'alésage à extrémité ouverte (10a) s'étendant longitudinalement le long de la tige tubulaire (10) ; et
une pluralité de bras élastiquement flexibles situés à l'extrémité distale de l'au moins une tige tubulaire (10), dans lequel au moins deux de la pluralité de bras élastiquement flexibles sont adaptés pour référencer des bords périphériques opposés de l'os à forer, dans lequel la pluralité de bras flexibles (55, 155) sont formés d'un plastique ou métal élastique et sont formés d'un seul tenant avec la tige tubulaire (10) de sorte que les bras élastiquement flexibles et la tige tubulaire (10) forment une construction monobloc fabriquée à partir de plastique ou métal élastique,
**caractérisé en ce que** le guide de forage (100) est configuré pour déterminer automatiquement un point central (C1, C2) d'un os (90, 91, 92, 93) à forer, le point central (C1, C2) étant équidistant des bords périphériques (P1, P2) de l'os.

2. Guide de forage (100) selon la revendication 1, dans lequel le guide de forage (100) est à positionnement automatique et peut en outre être à centrage automatique.

3. Guide de forage (100) selon la revendication 1, dans lequel l'os est une clavicule ou une coracoïde ou un péroné ou une rotule.

4. Guide de forage (100) selon la revendication 1, dans lequel la pluralité de bras flexibles (55, 155) est constituée de trois bras flexibles (55, 155) qui référencent les bords périphériques de deux os.

5. Guide de forage (100) selon la revendication 4, dans lequel les deux os sont une clavicule et une coracoïde.

6. Guide de forage (100) selon la revendication 4, dans lequel l'un parmi les trois bras flexibles (55, 155) référence un côté de l'os, et les deux autres parmi les trois bras flexibles référencent un autre os.

7. Guide de forage (100) selon la revendication 1, dans lequel au moins l'un de la pluralité de bras flexibles (55, 155) comprend un segment linéaire et un segment incurvé ou arqué, le segment incurvé ou arqué s'étendant au-delà d'une extrémité la plus distale de la tige tubulaire (10).

8. Guide de forage (100) selon la revendication 1, dans lequel la pluralité de bras flexibles (55, 155) est positionnée symétriquement par rapport à l'axe longitudinal.

9. Guide de forage (100) selon la revendication 1, comprenant deux tiges tubulaires (10) pour forer deux tunnels ou trous osseux dans l'os.
